(19) Europäisches Patentamt
European Patent Office
Office européen des brevets

(11) Publication number: **0 180 455 A2**

# (12) EUROPEAN PATENT APPLICATION

(21) Application number: **85307816.0**

(22) Date of filing: **29.10.85**

(51) Int. Cl.⁴: **C 07 C 93/04**

(30) Priority: **29.10.84 US 666006**

(43) Date of publication of application: **07.05.86**
**Bulletin 86/19**

(84) Designated Contracting States: **BE DE FR GB IT NL**

(71) Applicant: **ATLANTIC RICHFIELD COMPANY, 515 South Flower Street, Los Angeles California 90071 (US)**

(72) Inventor: **Kesling, Haven S., Jr., 248 Friendship Road, Drexel Hill Pennsylvania 19026 (US)**
Inventor: **Cooper, Charles F., 26 Wistar Road, Paoli Pennsylvania 19301 (US)**

(74) Representative: **Cropp, John Anthony David et al, MATHYS & SQUIRE 10 Fleet Street, London, EC4Y 1AY (GB)**

(54) **Process for the preparation of alkoxylated tertiary amine compounds.**

(57) Novel alkoxylated tertiary amine compounds of the formula

$$RO\,(R_1)_x\,CH_2\overset{\displaystyle R_2\atop|}{C}HNR_3R_4$$

are prepared by reacting an oxyalkylated alcohol with a secondary amine in the presence of a reductive amination catalyst at above ambient temperature and superatmospheric pressure. The amine compounds are useful as corrosion inhibitors, full additives, curing agents for epoxy resins and as biocides.

EP 0 180 455 A2

ACTORUM AG

BACKGROUND OF THE INVENTION

This application is related to a co-pending European application of ours and entitled Alkoxylated Tertiary Amine Compounds and filed on even date herewith, which application discloses certain aspects of the present invention.

In the above co-pending application which application is incorporated herein by reference, novel alkoxylated tertiary amine compounds are disclosed which are prepared by reacting an alcohol or mixtures of alcohols followed by catalytic amination of the resulting oxyalkylated alcohol with a secondary amine at desired temperatures and pressures to give the alkoxylated tertiary amine. The present invention is directed to a novel process for the preparation of the alkoxylated tertiary amines, such as the aminated propoxylated amines, disclosed in the above-mentioned copending application, which process is directed to the catalytic amination of an oxyalkylated alcohol with certain secondary amine compounds. The alkoxylated tertiary amines of the present invention are useful, for example, as biocides, fuel additives and corrosion inhibitors.

Alkoxylated amine compounds of various structures and processes for their preparation are well known. A representative reference patent is U. S. Patent 3,236,895 which describes polyamines prepared by reacting polyols with alkylsulfonyl halides and treating the products with

In an article by E. J. Schwolgler and H. Adkins, Journal Am. Chem. Society, Vol. 61, pp 3499-3502 (1939) certain tertiary amines prepared from primary alcohols and secondary amines using hydrogenation and dehydrogenation catalysts have been reported. Early assertions that Raney Nickel could be used in such a reaction is contradicted by U. S. Patent 4,138,437 describing copper chromite as the only catalyst capable of reducing transalkylation reactions resulting in a amine product mixture. U. S. Patent 4,442,306 shows a process for the preparation of tertiary amines using a copper formate catalyst. U. S. Patent Numbers 4,404,403; 4,404,404, and 4,409,399 describe a reaction of primary alcohols and dimethylamine using copper oxide or copper hydroxide catalysts in conjunction with Group IIA metals, particularly nickel to prepare tertiary amines.

In the area of amination of oxyalkylane oligomers, U. S. Patent Numbers 3,847,992 and 3,654,370 describe processes for preparing polyoxyalkylene polyamines using ammonia and a Raney-Nickel catalyst or a catalyst of nickel, copper and chromium as shown in U. S. Patent 3,152,998. The amination process using ammonia has also been applied to certain specific polypropylene glycols used as defoamers as shown in U. S. Patent 4,075,130 as well as to propoxylated butane diols as described in U. S. Patent 4,181,682. Propoxylated alcohols have been aminated with ammonia to prepare primary amines as intermediates in the preparation of other products as shown in U. S. Patent Nos. 4,107,096; 4,261,845 and 4,332,595.

Applicants are not aware of any truly pertinent prior art that is deemed to be anticipatory or suggestive

of the process for the preparation of the tertiary amine compounds by aminating an oxyalkylated alcohol with a secondary amine.

It is therefore an object of this invention to provide a novel method for the preparation of alkoxylated tertiary amine compounds.

These and other objects and advantages of this invention will become apparent from the description of the invention which follows and from the claims.

DESCRIPTION OF THE INVENTION

The alkoxylated tertiary amine compounds prepared by the process of the present invention have the general formula:

$$RO\,(R_1)_x\,CH_2\overset{\displaystyle R_2}{\overset{|}{C}}HNR_3R_4$$

wherein R is a straight or branched chain alkyl group having from 1 to 11, preferably from 6 to 10 carbon atoms, a cyclic alkyl group having from 5 to 10 carbon atoms, preferably from 5 to 6 carbon atoms, an aryl group having up to 12 carbon atoms, preferably 6 to 10 carbon atoms, or an aralkyl or alkaryl group having up to 18 carbon atoms, preferably 7 to 12 carbon atoms, $R_1$ is a single unit or a series of units of the formula:

$$\leftarrow CH_2\overset{\displaystyle R_5}{\overset{|}{C}}HO\rightarrow_x$$

wherein in each unit $R_5$ is independently selected from the group consisting of hydrogen and a straight or branched chain alkyl group having from 1 to 12 carbon atoms, preferably 1 to 4 carbon atoms, and x is an integer of from 0 to 40, preferably from 3 to 20, $R_2$ is hydrogen or a

straight or branched chain alkyl group having from 1 to 12 carbon atoms, preferably 1 to 4 carbon atoms, and $R_3$ and $R_4$ are each independently selected from the group consisting of straight and branched chain alkyl groups having from 1 to 12 carbon atoms, preferably 1 to 8 carbon atoms, cyclic alkyl groups having from 5 to 10 carbon atoms, preferably 5 to 6, 1 to 4 carbon atom alkyl substituted or unsubstituted benzyl groups and allyl.

The alkoxylated tertiary amine compounds are prepared by reacting an alcohol or mixtures of alcohols with an alkylene oxide to prepare an oxyalkylated alcohol followed by catalytic amination of the oxyalkylated alcohol with a secondary amine such as a dialkyl, dicyclic alkyl, dibenzyl or diallyl amine under increased temperatures and pressure.

The oxyalkylation reaction is conducted by methods well known in the art by reacting an appropriate alcohol such as, for example, methanol, etc. or the short chain plasticizer range linear Ziegler type alcohols containing various mixtures of $C_6$ through $C_{10}$ alcohols with an alkylene oxide, such as propylene oxide in the presence of an alkali metal or alkaline earth catalyst as taught, for example in the U. S. Patent Nos. 2,508,035; 2,617,830; 2,671,115 and 3,382,285 and incorporated herein by reference. Typical of such Ziegler alcohols, which are also employed in the instant invention, are the "EPAL 810 or 610" and "ALFOL 810 or 610" types which are mixtures of predominately $C_8$ and $C_{10}$ straight chain alcohols sold commercially, for example, by the Ethyl Corporation and the Continental Oil Company

respectively.

The alcohols which may be reacted with the alkylene oxides to prepare the oxyalkylated alcohols for further processing include any aliphatic or branched monohydric alcohol containing from 1 to 11 carbon atoms, cyclic alcohols, aryl alcohols as well as aralkyl and alkaryl alcohols as defined by R in the above formula. Representative alcohols include, for example, methyl, ethyl, n-iso-sec- and tert-butyl, amyl, hexyl, octyl, nonyl, n- and isopropyl, decyl, and benzyl alcohols, cyclohexanol, 2-ethyl hexanol, methyl cyclohexanol, 3-methyl butanol, 1-heptanol and the like or mixtures thereof.

The alkylene oxides which may be employed to prepare the oxyalkylated alcohols include, for example, ethylene and propylene oxide and the higher alkylene oxides, i.e., containing from 3 to 12 carbon atoms. Preferred among the oxides is propylene oxide. Illustrative higher alkylene oxides include, for example, isobutylene oxide, 1,2-epoxybutane, 2,3-epoxybutane, 2,3-dimethyl-2,3-epoxybutane, 2-methyl-2,3-epoxypentane, 1,2-epoxypentane, 5-methyl-1,2-epoxyheptane, 1,2-epoxyoctane, 2,4,4-trimethyl-1,2-epoxypentane, 2,4,4-trimethyl-2,3-epoxypentane, 1,2-epoxynonane, 1,2-epoxydecane, 1,2-epoxydodecane, 3-ethoxy-4-propyl-3,4-epoxyheptane, and the like or mixtures thereof. In general, the oxyalkylated alcohol formation with, for example propylene oxide, is carried out at temperatures in the range of from about 70°C to 150°C preferably 90°C to 100°C under moderately elevated pressures of from about atmospheric to 500 psig, preferably from 50 to 100 psig, and in the presence of between about 0.01 to 1, preferably

0.2 to 0.3 weight % of total reactants of an alkaline-reacting material or catalyst such as sodium, potassium, calcium, barium and strontium hydroxides. In general a controlled amount of an alkylene oxide or admixture thereof is slowly contacted with the alcohol reactant for a period ranging up to about 20 hours in an amount sufficient to prepare the desired oxyalkylated reaction product mixture. On completion of the oxyalkylation reaction, the crude reaction product containing the catalyst is normally treated with an inorganic acid or acid-forming material to neutralise the reaction product after which it is filtered to produce a finished oxyalkylated product.

If desired the oxyalkylation of the alcohol may be carried out in the presence of a suitable inert solvent such as toluene, benzene, xylene, tetrahydrofuran, heptane, hexane, pentane, octane, etc. which are free of water.

Preparation of the alkoxylated tertiary amine involves the novel process of aminating the oxyalkylated alcohol with a secondary amine in the presence of from about 0.5 to 20 weight %, preferably 2.5 to 10 weight % of amination catalyst based on the total reaction mixture. Any amination catalyst known in the art may be employed, such as, for example, Raney Nickel, supported noble metals, and catalysts containing Cu, Cr and promoters such as nickel; however, catalysts containing CuO and $Cr_2O_3$ in various ratios, Cu-Cr supported on magnesium aluminate spinel and ruthenium on activated carbon are preferred catalysts.

The secondary amines which may be used to aminate the oxyalkylated alcohol include, for example,

dimethylamine, diethylamine, di-n-propylamine, di-i-propylamine, di-n-butylamine, di-n-amylamine, di-n-hexylamine, di-n-octylamine, di-nonylamine, di-n-decylamine, di-i-octylamine, di-benzylamine, dimethylbenzylamine, diethylbenzylamine, dicyclohexylamine, dicyclopentylamine, and diallylamine.

The amination reaction of oxyalkylated alcohols employing secondary amines is conducted at a temperature within the range of from about 150°C to 325°C with a preferred range being from 175°C to 275°C. The pressure may be varied from about 50 psig to 30000 psig with the preferred range being 100 psig to 250 psig.

Although not required, solvents, if desired, which are chemically inert to the components of the reaction system may be employed in the reductive amination reaction. Suitable solvents include, for example, heptane, pentane, cyclohexane, benzene, toluene, xylene, etc.

The reaction time is generally dependent upon the oxyalkylated alcohol and secondary amine being reacted, temperature and pressure and on the amount of catalyst employed as well as the type of equipment being employed. Reaction times will vary dependent on whether the process is continuous, semi-continuous or batch.

A general postulated equation for the reaction of the present invention may be represented as follows:

$$\underset{\text{oxyalkylated alcohol}}{RO(R_1)_xCH_2\overset{\overset{R_2}{|}}{C}HOH} \quad + \quad \underset{\text{amine}}{HNR_3R_4} \quad \xrightarrow[\Delta\text{ + pressure}]{\text{catalyst}}$$

$$\underset{\text{alkoxylated tertiary amine}}{RO\ (R_1)_x\ CH_2\overset{\overset{R_2}{|}}{C}HNR_3R_4} \quad + \quad H_2O$$

0180455

wherein R, $R_1$, $R_2$, $R_3$, $R_4$ and x are as herein above described. A wide variety of alkoxylated amines can be prepared by the process of this invention.

The following Examples are provided to illustrate the process of the present invention in accordance with the principles of this invention, but are not to be construed as limiting the invention in any way except as indicated by the appended claims.

EXAMPLE 1

Preparation of Oxyalkylated Alcohols - General

All oxyalkylation reactions were carried out in a 2 gallon stirred stainless steel autoclave. A 0.25 percent catalyst solution was prepared using the desired alcohol admixture and potassium hydroxide by preheating at 75-100°C. Water was removed under vacuum and the catalyst solution was charged hot under dry nitrogen into the autoclave. The remaining alcohol was added to the reactor followed by a purge with dry nitrogen. The reactor was heated to 95°C and dry alkylene oxide in the appropriate stoichiometry was slowly added over a period of from 24 to 48 hours using a pressure demand control valve system to control the addition rate. A reference pressure was set at 60 psig and if the reactor pressure dropped below the reference pressure the control valve opened and more alkylene oxide was charged to the reactor. When the pressure increased to greater than 60 psig, the valve closed. The alkylene oxide was contained in a hoke that was suspended on a weight load cell, thereby permitting the charging of the correct amount of alkylene oxide. Since the hoke had an 80 psig nitrogen pressure head, the overall reactor

pressure increased to 80 psig when all the liquid alkylene oxide was pushed out of the load cell hoke into the reactor. When the reaction was complete the product was removed hot from the reactor and was treated with magnesium silicate (4 grams per 250 grams of product) for 2 hours at 120°C in order to remove the catalyst. The resulting product was vacuum filtered through a bed of diatomaceous earth at 60-80°C to provide the pure oligomeric polyol. Hydroxyl number, VPO molecular weight, GPC analyses and $^{13}C$ NMR were employed to determine and characterize the product oligomers. In general, for the $C_6$-$C_{10}$ plasticizer range alcohols and other short chain alcohols, sufficient alkylene oxide is employed in the oxyalkylation reaction to effect preparation of an oxyalkylated derivative admixture, having an average number of alkylene oxide units per molecule in the product of between 3 and 40, preferably between 5 and 20.

EXAMPLE 2

Amination Catalyst Preparation

One of the catalysts used to prepare the alkoxylated tertiary amines of the invention employed magnesium aluminate spinel as a base support material. The catalyst was composed of a magnesium aluminate spinel containing 20-25 weight % based on the weight of the catalyst of copper oxide (CuO) and 1.0-2.0 weight %, based on the total weight of the catalyst, of chromium trioxide ($Cr_2O_3$), which typically was prepared by dissolving 337.5 gram of aluminum nitrate ($Al(NO_3)_3 \cdot 9H_2O$) in 800 ml. of water at ambient temperature and pressure and dissolving 115.1 grams of magnesium nitrate ($Mg(NO_3)_2 \cdot 6H_2O$) in 800 ml. of water. The two solutions were combined with stirring and then slowly

added to 500 ml of water in a sitrred mixing tank while adding 1.35 liters of concentrated ammonium hydroxide to the mixing tank at a rate sufficient to maintain a pH of 10.0 to 12.0. The resulting precipitate was filtered followed by washing with 1 liter of distilled water in 500 ml intervals. The wet cake of catalyst was dried at 120°C and sieved to 10 mesh followed by calcination overnight at 500°C. The calcinated base material was impregnated with 32.8 mls of an aqueous solution containing copper nitrate equivalent to 11.4 grams copper oxide and chromium nitrate equivalent to .52 grams of chromite trioxide. The impregnated catalyst was dried at 120°C and calcined in air for one (1) hour at 400°C. The resulting catalyst contained 22 weight % copper oxide and 1 weight % chromium trioxide on the magnesium aluminate spinel base support.

EXAMPLE 3

Amination of Alkoxylated Alcohols

100 CC. of the catalyst prepared according to Example 2 was packed into a 36" by 1" stainless steel fixed bed reactor. The catalyst was prereduced with hydrogen at 250°C prior to use in the amination reaction. Evolution of water from the reaction was used to follow the amination process. Hydrogen, dialkylamine, and alkoxylated oligomer alcohol were fed to the reactor at a 5:2:1 molar ratio using .75-2.0 hourly space velocity at a 225°C reaction temperature. The gases leaving the reactor were passed through a heated transfer line to a separator to disengage the alkoxylated tertiary amine product. $^{13}C$ NMR of a typical reaction mixture, which utilized the "EPAL 810"

($C_8$-$C_{10}$-[PO]$_8$OH) alkoxylated oligomer alcohol in the feed indicated a 95% conversion. Selectivity to the alkoxylated tertiary amine was 95%, other by-products were 4% alkoxylated secondary amine ("EPAL" $C_8$-$C_{10}$-O[PO]$_8$N ($CH_3$)H and 1% alkoxylated primary amine ("EPAL" $C_8$-$C_{10}$-O [PO]$_8$ $NH_2$.

## EXAMPLES 4-38

In Examples 4-38 which follow in table form, the batch reaction amination procedure of Example 3 was repeated using various oxyalkylated alcohols and secondary amine reactants. The reactions were carried out in a stirred 300 CC stainless steel autoclave for a period of from 4 to 8 hours under a continuous hydrogen flow of from 300 to 600 ml/min. The oxyalkylated oligomers (.20 moles) were charged to the reactor followed by heating to the reaction temperature indicated under a continuous flow of hydrogen. The secondary amine feed was initiated, once temperature was obtained, at a rate of .23 moles/hour. In Examples 4 to 26 and Examples 33 to 38, 5 weight percent of a catalyst of 80% CuO and 20% $Cr_2O_3$ was employed. Examples 27 and 28 employed 2.5 wt.% and 10 wt.% of the same catalyst respectively. Example 29 used 5 wt.% of a catalyst of 70% CuO and 30% $Cr_2O_3$. Example 30 used 5 wt.% of a catalyst of 60% CuO and 40% $Cr_2O_3$. Example 31 used 5 wt.% of copper chromite supported on magnesium aluminate spinel and in Example 32 a catalyst of 10% ruthenium on activated carbon was employed. The products were analyzed by NMR and titration methods to determine % conversion and % selectivity to the alkoxylated tertiary amine and byproduct primary and secondary amines.

The reaction conditions and reactants according

to the following equation are set forth in Table 1.

$$RO(R_1)_xCH_2\overset{\overset{\displaystyle R_2}{|}}{C}HOH + HNR_3R_4 \longrightarrow$$

$$RO(R_1)_xCH_2\overset{\overset{\displaystyle R2}{|}}{C}NR_3R_4 + H_2O$$

wherein R, $R_1$, $R_2$, $R_3$, $R_4$ and x are as above described. The results of the reactions showing % conversion and selectivity are summarized in Table 2.

**TABLE 1**

| Ex. No. | R | $R_5$ | $R_2$ | $R_3$ | $R_4$ | X | Temp (°C) | Pressure (psig) |
|---|---|---|---|---|---|---|---|---|
| 4 | "EPAL" 810 | $CH_3$ | $CH_3$ | $CH_3$ | $CH_3$ | 7 | 270 | 150 |
| 5 | "EPAL" 610 | $CH_3$ | $CH_3$ | $CH_3$ | $CH_3$ | 7 | 270 | 150 |
| 6 | "EPAL" 810 | $CH_3$ | $CH_3$ | $CH_3$ | $CH_3$ | 7 | 270 | 150 |
| 7 | "EPAL" 610 | $CH_3$ | $CH_3$ | $CH_3$ | $CH_3$ | 7 | 270 | 150 |
| 8 | "EPAL" 810 | $CH_3$ | $CH_3$ | $CH_3$ | $CH_3$ | 7 | 270 | 150 |
| 9 | "EPAL" 810 | $CH_3$ | $CH_3$ | $CH_3$ | $CH_3$ | 7 | 200 | 150 |
| 10 | "EPAL" 810 | $CH_3$ | $CH_3$ | $CH_3$ | $CH_3$ | 7 | 225 | 150 |
| 11 | "EPAL" 810 | $CH_3$ | $CH_3$ | $CH_3$ | $CH_3$ | 7 | 300 | 150 |
| 12 | "EPAL" 810 | $CH_3$ | $CH_3$ | $CH_3$ | $CH_3$ | 2 | 270 | 150 |
| 13 | "EPAL" 810 | $CH_3$ | $CH_3$ | $CH_3$ | $CH_3$ | 4 | 270 | 150 |
| 14 | "EPAL" 810 | $CH_3$ | $CH_3$ | $CH_3$ | $CH_3$ | 11 | 250 | 150 |
| 15 | "EPAL" 810 | $CH_3$ | $CH_3$ | $CH_3$ | $CH_3$ | 15 | 270 | 150 |
| 16 | "EPAL" 810 | $CH_3$ | $CH_3$ | Ethyl | Ethyl | 7 | 275 | 50 |
| 17 | "EPAL" 810 | $CH_3$ | $CH_3$ | 2-propyl | 2-propyl | 7 | 275 | 150 |
| 18 | "EPAL" 810 | $CH_3$ | $CH_3$ | n-butyl | n-butyl | 7 | 275 | 150 |
| 19 | "EPAL" 810 | $CH_3$ | $CH_3$ | n-octyl | n-octyl | 7 | 275 | 100 |
| 20 | "EPAL" 810 | $CH_3$ | $CH_3$ | allyl | allyl | 7 | 275 | 100 |

TABLE 1 (Cont'd)

| Ex. No. | R | $R_5$ | $R_2$ | $R_3$ | $R_4$ | X | Temp (°C) | Pressure (psig) |
|---|---|---|---|---|---|---|---|---|
| 21 | "EPAL" 810 | $CH_3$ | $CH_3$ | benzyl | benzyl | 7 | 275 | 100 |
| 22 | "EPAL" 810 | $CH_3$ | $CH_3$ | $CH_3$ | $CH_3$ | 24 | 275 | 100 |
| 23 | octyl | $CH_3$ | $CH_3$ | $CH_3$ | $CH_3$ | 19 | 275 | 100 |
| 24 | decyl | $CH_3$ | $CH_3$ | $CH_3$ | $CH_3$ | 9 | 275 | 100 |
| 25 | 2-ethylhexyl | $CH_3$ | $CH_3$ | $CH_3$ | $CH_3$ | 7 | 275 | 100 |
| 26 | 2-ethylhexyl | $CH_3$ | $CH_3$ | $CH_3$ | $CH_3$ | 11 | 275 | 100 |
| 27 | hexyl | $CH_3$ | $CH_3$ | $CH_3$ | $CH_3$ | 19 | 275 | 100 |
| 28 | 2-ethylhexyl | $CH_3$ | $CH_3$ | $CH_3$ | $CH_3$ | 4 | 275 | 100 |
| 29 | "EPAL" 810 | $CH_3$ | $CH_3$ | $CH_3$ | $CH_3$ | 7 | 275 | 100 |
| 30 | "EPAL" 810 | $CH_3$ | $CH_3$ | $CH_3$ | $CH_3$ | 7 | 275 | 100 |
| 31 | "EPAL" 810 | $CH_3$ | $CH_3$ | $CH_3$ | $CH_3$ | 7 | 275 | 100 |
| 32 | "EPAL" 810 | $CH_3$ | $CH_3$ | $CH_3$ | $CH_3$ | 7 | 275 | 100 |
| 33 | "EPAL" 810 | $CH_3$ | ethyl | $CH_3$ | $CH_3$ | 8 | 275 | 100 |
| 34 | "EPAL" 810 | $8CH_3+3H$ (Random) | H | $CH_3$ | $CH_3$ | 8 | 275 | 100 |
| 35 | "EPAL" 810 | $4H_2+7CH_3$ (block) | $CH_3$ | $CH_3$ | $CH_3$ | 4 | 275 | 100 |
| 36 | "EPAL" 810 | ethyl | ethyl | $CH_3$ | $CH_3$ | 2 | 275 | 100 |
| 37 | "EPAL" 810 | 1 butyl $2CH_3$ (block) | $CH_3$ | $CH_3$ | $CH_3$ | 1 | 275 | 100 |
| 38 | "EPAL" 810 | 1 octyl $2CH_3$ (block) | $CH_3$ | $CH_3$ | $CH_3$ | 1 | 275 | 100 |

TABLE 2

| Ex. No. | % Conversion | % Selectivity Primary | Secondary | Tert-Amine |
|---|---|---|---|---|
| 4 | 99 | 1 | 9 | 90 |
| 5 | 100 | 0 | 8 | 92 |
| 6 | 99 | 1 | 10 | 89 |
| 7 | 99 | 0 | 5 | 95 |
| 8 | 91 | 0 | 3 | 97 |
| 9 | 72 | 0 | 8 | 92 |
| 10 | 83 | 0 | 6 | 94 |
| 11 | 100 | 3 | 10 | 87 |
| 12 | 99 | 0 | 4 | 96 |
| 13 | 100 | 1 | 7 | 92 |
| 14 | 98 | 1 | 11 | 88 |
| 15 | 99 | 0 | 10 | 90 |
| 16 | 95 | 0 | 11 | 89 |
| 17 | 100 | 1 | 7 | 92 |
| 18 | 99 | 0 | 10 | 90 |
| 19 | 99 | 0 | 9 | 91 |
| 20 | 99 | 0 | 12 | 88 |
| 21 | 99 | 0 | 8 | 92 |
| 22 | 100 | 0 | 15 | 85 |
| 23 | 100 | 1 | 8 | 91 |
| 24 | 100 | 0 | 10 | 90 |
| 25 | 99 | 0 | 10 | 90 |
| 26 | 99 | 0 | 9 | 90 |
| 27 | 80 | 0 | 2 | 98 |
| 28 | 100 | 5 | 20 | 75 |
| 29 | 100 | 3 | 15 | 82 |
| 30 | 99 | 5 | 25 | 70 |
| 31 | 100 | 0 | 2 | 98 |
| 32 | 99 | 11 | 28 | 61 |
| 33 | 100 | 1 | 10 | 89 |
| 34 | 99 | 0 | 8 | 92 |
| 35 | 100 | 0 | 12 | 88 |
| 36 | 100 | 0 | 9 | 91 |
| 37 | 100 | 0 | 10 | 90 |
| 38 | 100 | 0 | 4 | 96 |

**EXAMPLE 39 .**

In a 2 gallon batch stirred stainless steel autoclave was charged 200 grams of 150 mesh of copper chromite catalyst (containing 41 mole % copper, 31 mole % chromium and .3 mole % barium) along with 5000 grams of "EPAL" 810-$C_8$-$C_{10}$-O [PO]$_8$OH alkoxylated oligomer alcohol. Stirring and heating was initiated followed by heating to 175° under nitrogen. Hydrogen gas (10 liters/hour) was fed into the reactor under 50 psig back pressure. Dimethylamine (5 mole %) in the hydrogen stream was fed continuously to the reactor (one reactor volume turnover per hour) over a six hour period while holding the reactor temperature at 270°C. Conversion of the alkoxylated alcohol was 99% and selectivity to the alkoxylated tertiary amine 97.6%.

0180455

**WE CLAIM:**

1. A process for the preparation of an alkoxylated tertiary amine having the formula

$$RO(R_1)_x\ CH_2\overset{\overset{\large R_2}{|}}{C}HNR_3R_4$$

or a mixture of such amines which comprises reacting an oxyalkated alcohol of the formula

$$RO(R_1)_x\ CH_2\overset{\overset{\large R_2}{|}}{C}HOH$$

or a mixture of such alcohols, with a secondary amine of the formula $HNR_3R_4$ at a temperature in the range of from about 150°C to 325°C and a pressure of from about 50 psig to 3000 psig in the presence of from 0.5 to 20 weight percent of an amination catalyst based on the total reaction mixture and wherein R is a straight or branched chain alkyl group having from 1 to 11 carbon atoms, a cyclic alkyl group having from 5 to 10 carbon atoms, an aryl group having up to 12 carbon atoms, or an aralkyl or alkaryl group having up to 18 carbon atoms, $R_1$ is a single unit or a series of units of the formula:

$$\leftarrow CH_2\overset{\overset{\large R_5}{|}}{C}HO \rightarrow_x$$

wherein in each unit $R_5$ is independently selected from the group consisting of hydrogen and a straight or branched chain alkyl group having from 1 to 12 carbon atoms and x is an integer of from 0 to 40, $R_2$ is hydrogen or a straight or branched chain alkyl group having from 1 to 12 carbon atoms, $R_3$ and $R_4$ are each independently selected from the group consisting of straight and branched chain alkyl groups having from 1 to 12 carbon atoms cyclic alkyl groups having from 5 to 10 carbon atoms, 1 to 4 carbon

atom alkyl substituted or unsubstituted benzyl groups and allyl.

2. A process according to claim 1 wherein R is a straight chain alkyl group having from 8 to 10 carbon atoms, $R_1$ is a

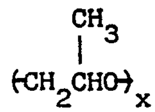 group with x being 7 and $R_2$, $R_3$ and $R_4$ are $CH_3$ groups.

3. A process according to claim 2 but wherein $R_1$ is a

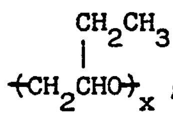 group, a 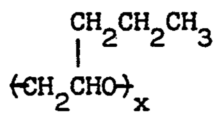 group or a

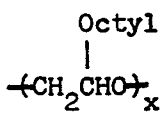 group.

4. A process according to any one of claims 1 to 3 wherein the amination catalyst is a mixture of CuO and $Cr_2O_3$.

5. A process according to any one of claims 1 to 3 wherein the amination catalyst is copper and chromium supported on magnesium aluminate spinel.

6. A process according to any one of claims 1 to 3 wherein the amination catalyst is ruthenium supported on activated carbon.

7. A process according to any one of claims 1 to 6 wherein the amination catalyst is employed in an amount of from 2.5 to 10 weight percent.

8. A process according to any one of claims 1 to 7 wherein the temperature is in the range of from 175°C to 275°C.

9. A process according to any one of claims 1 to 8 wherein the pressure is from 100 psig to 250 psig.